# EUROPÄISCHE PATENTSCHRIFT

(11) **EP 2 030 596 B1**
(45) Veröffentlichungstag und Bekanntmachung des Hinweises auf die Patenterteilung: **27.11.2019**
(21) Anmeldenummer: 07115055.1
(22) Anmeldetag: 27.08.2007
(51) Int. Cl.: A61B 17/80, A61F 2/30, A61F 2/28

(54) **Implantat zur Behandlung von Knochen.**
Implant for treating bones.
Implant destiné au traitement d'os.

(43) Veröffentlichungstag der Anmeldung: 04.03.2009
(73) Patentinhaber: Medartis Holding AG, 4057 Basel (CH)
(72) Erfinder: Mullis, Andreas, 4450, Sissach (CH); Breitenstein, Michael, 4493, Wenslingen (CH); Lieger, Oliver, 6045 Meggen (CH)
(74) Vertreter: Hepp Wenger Ryffel AG

(56) Entgegenhaltungen:
- EP-A- 0 654 250
- WO-A-2004/093657
- DE-U1- 29 913 334
- US-A- 5 139 497
- US-A- 5 383 931
- US-A- 5 743 913
- US-A- 6 030 218
- US-A1- 2005 192 675
- US-B2- 6 394 807

## Beschreibung

Die Erfindung betrifft ein Implantat zur Behandlung von Knochen gemäss dem Oberbegriff des unabhängigen Patentanspruchs. Derartige Implantate können etwa zur Abdeckung von Knochendefekten oder -fehlbildungen oder von Bohrlöchern verwendet werden. Weiterhin können sie bei einer Knochenrekonstruktion eingesetzt werden. Insbesondere betrifft die Erfindung ein Implantat für eine Orbita, also eine knöcherne Augenhöhle. Bei einer gezielten, punktuellen, von vorne kommenden Gewalteinwirkung auf einen menschlichen oder tierischen Augapfel kann es zu einer Fraktur der Orbita kommen. Weiter können Fehlbildungen und Tumore ähnliche Auswirkungen haben. Infolgedessen wird der Augapfel nicht in der anatomisch korrekten Form gehalten, so dass die Gefahr einer Einklemmung der Augenweichteile und einer Fehlsichtigkeit besteht. Derartige Defekte können mit Hilfe von Implantaten behandelt werden, die an dem/den defekten Knochen oder am benachbarten Knochenmaterial fixiert werden. Aufgrund der filigranen Struktur der Orbita, im Speziellen des Orbitabodens, und der individuellen Anatomie stellen sich der Medizintechnik hierbei besondere Herausforderungen. Insbesondere sollte die Struktur des Implantats so beschaffen sein, dass es sich intraoperativ, einfach und möglichst genau an die Anatomie der defekten Orbita anpassen lässt. Überdies sollte es im angebogenen Zustand rigide sein, damit es die Funktion des defekten Knochens übernehmen kann und dabei den Augapfel in der anatomisch korrekten Lage halten.

Aus US 5,139,497 ist ein derartiges Implantat bekannt, welches ein längliches Basiselement sowie drei davon nach hinten abstehende Blätter aufweist. Das mittlere der Blätter ist dabei für den Orbitaboden vorgesehen, die beiden äusseren für die Seitenwände der Augenhöhle. Jedes der drei Blätter ist in einem Innenbereich durchgehend mit Perforationen versehen. Ein wesentlicher Nachteil dieses Implantats ist, dass sich aufgrund der Geometrie der Perforationen die einzelnen Blätter nur schwer an die Anatomie der Orbita anpassen lassen.

Weiterhin beschreibt US 5,743,913 eine Gitterplatte, welche ebenfalls ihren Einsatz in der Orbitabodenchirurgie finden kann. Die Gitterplatte verfügt im gesamten Innenbereich über hantelförmige Aussparungen, welche von bogenförmigen Stegen begrenzt sind. Diese Gitterplatte hat unter anderem den Nachteil, dass die Gitterstruktur derart grossmaschig ist, dass beim Anbiegen der Struktur keine stufenlose Freiformfläche entsteht. Im Gegenteil bilden sich unerwünscht Absätze in der Fläche, welche das Implantieren des Implantats erschweren. Eine blosse Verringerung der Maschengrösse mit ebenfalls zwingender Verringerung der Plattendicke (damit eine vergleichbare Anformbarkeit und Deformierbarkeit gewährleistet werden kann), welche diesem Nachteil Abhilfe verschaffen würde, würde dagegen zu einer geringeren Tragfähigkeit des Implantates bzw. der Unterstützung des Augapfels führen.

Das Dokument US 2006/224242 A1, aus dem der Oberbegriff des unabhängigen Anspruchs abgeleitet wird, offenbart flächige, biegbare Implantate, die ein Polymermaterial und ein darin eingebettetes Metallgitter aufweisen. Die Implantate können ferner Befestigungslaschen zum Befestigen des Implantats an einem Knochen enthalten.

Es ist die Aufgabe der vorliegenden Erfindung, die Nachteile der bekannten Implantate zu überwinden und insbesondere ein Implantat, insbesondere für die Orbita bereitzustellen, welches flexibel und individuell anpassbar ist und gleichzeitig in einem insbesondere dreidimensional deformierten Zustand genügend rigide ist, um ein Verschieben oder Verziehen zu verhindern. Darüber hinaus sollte das Implantat in der Lage sein, während einer längeren Zeit oder sogar ein Menschenleben lang eine tragende Funktion zu übernehmen. Weiterhin soll ein Implantat geschaffen werden, welches flexibel einsetzbar ist und beispielsweise wahlweise für die mediale Wand, die laterale Wand oder den Orbitaboden verwendet werden kann. Andererseits sollte das Implantat wahlweise in ein linkes oder ein rechtes Auge einsetzbar sein.

Die Erfindung betrifft ein Implantat zur Behandlung von Knochenfrakturen mit einer Stützstruktur und mindestens einem Anpassungsbereich, der insbesondere flächig ausgebildet ist. Unter einem Implantat wird somit ein Gegenstand verstanden, welcher für die Chirurgie und insbesondere die Knochenchirurgie geeignet ist. Zu diesem Zweck sollte es unter anderem die Erfordernisse der einschlägigen Gesetze und Normen erfüllen, wie etwa des deutschen Medizinproduktegesetzes, der deutschen Medizinprodukte-Verordnung, der EU-Richtlinie 93/42/EWG über Medizinprodukte, des CFR (Code of Federal Regulations) 21 in den USA oder der ISO 13485:2003.

Das Implantat ist an die individuelle Anatomie etwa einer Orbita anpassbar. Bevorzugt ist es hierzu insbesondere dreidimensional deformierbar. Die Anpassungsbereiche sind dreidimensional formbar. Hingegen verleiht die Stützstruktur dem Implantat die erforderliche Steifigkeit, schränkt aber eine gewünschte Deformation des Implantats nicht ein. Auf diese Weise wird ein ausgezeichneter Kompromiss zwischen einer Anformbarkeit und einer Steifigkeit erzielt.

Erfindungsgemäss weist die Stützstruktur einen Innenbereich auf, der innerhalb eines Randes des Implantats angeordnet ist. Somit liegt die Stützstruktur insbesondere nicht ausschliesslich im Randbereich des Implantats. Ebenfalls erfindungsgemäss ist der Anpassungsbereich plastisch deformierbar.

Im Gegensatz zum bekannten Stand der Technik, bei dem eine Stützstruktur lediglich im Randbereich des Implantats vorgesehen ist und ein regelmässig perforierter Gitterbereich die gesamte innere Fläche ausfüllt, ergibt sich somit eine wesentlich erhöhte Stabilität bei gleichzeitiger kleiner Baugrösse der Elemente der einzelnen Anpassungsbereiche und dreidimensionaler Deformierbarkeit. Insbesondere ist das erfindungsgemässe Implantat auch im dreidimensional deformierten Zustand genügend rigide und daher weniger anfällig gegenüber unerwünschten weiteren Deformationen. Ein derartiges Implantat ist folglich insbesondere zur Behandlung einer Fraktur einer Orbita geeignet.

Die Stützstruktur liegt nicht ausschliesslich am Rand vor und kann deshalb gezielt in den Bereichen vorgesehen werden, in denen erfahrungsgemäss eine hohe Steifigkeit gefordert ist. Bevorzugt umfasst das Implantat ein biokompatibles Material. Dabei kann es sich beispielsweise um einen metallischen Werkstoff wie Titan handeln. Alternativ oder zusätzlich kann das Implantat ein biokompatibles polymeres oder polymer-keramisches Material enthalten. Derartige Materialien sind etwa in dem US-Patent US 5,383,931 beschrieben.

Bei den Anpassungsbereichen handelt es sich bevorzugt um Gitterbereiche, deren Struktur an sich aus dem Stand der Technik bekannt ist (etwa aus den oben genannte Dokumenten US 5,139,497 und US 5,743,913 sowie aus WO 03/007831, WO 2005/089096, DE 19507544, US 5,814,048 oder US 5,468,242). Die Struktur der Gitterbereiche ist durch die Erfindung in keiner Weise eingeschränkt. Die Gitterbereiche können etwa eine im Wesentlichen periodische Struktur aufweisen. Alternativ können sie auch eine unregelmässige Struktur besitzen. Sie können mehrere Stege umfassen, die gerade und/oder gebogen sind. Gebogene Gitterstege sind dabei besonders bevorzugt, da sie die Gitterbereiche leicht auch dreidimensional deformierbar machen. Alternativ hierzu kann es sich bei den Anpassungsbereichen jedoch auch um Membranen oder andere flächige geeignete Gebilde handeln.

Gemäss einer bevorzugten Ausführungsform umfasst der Innenbereich einen Zentralbereich, welcher sich im Zentrum des Implantats befindet. Bevorzugt erstreckt sich von diesem Zentralbereich mindestens ein Stützsteg in Richtung des Randes des Implantats. Vorteilhaft weist die Stützstruktur mindestens drei solcher Stützstege auf, welche etwa sternförmig um den Zentralbereich angeordnet sind. Bevorzugt erstrecken sich die Stützstege bis zum Rand des Implantats. Es ist jedoch auch denkbar, dass sie im Inneren des Implantats enden.

Gemäss einer Weiterbildung weist die Stützstruktur zusätzlich Randbereiche auf, welche am Rand des Implantats angeordnet sind. Eine Ausführung sieht vor, dass der Randbereich das Implantat vollständig umschliesst und somit den kompletten Rand bildet. Alternativ dazu kann der Randbereich nur einen Teil des Randes bilden; in diesem Falle wird der verbleibende Teil des Randes etwa von den Rändern der Anpassungsbereiche gebildet.

In einer weiteren bevorzugten Ausführungsform weist das Implantat mindestens zwei Anpassungsbereiche auf, welche durch einen Teil des Innenbereichs der Stützstruktur räumlich voneinander getrennt sind. In diesem Fall werden die Anpassungsbereiche von der Stützstruktur zusammengehalten. Dies ermöglicht eine besonders flache Bauform des Implantats, da die Anpassungsbereiche und die Stützstruktur in einer einzigen ebenen oder gekrümmten Fläche mit geringer Dicke verlaufen können.

Alternativ hierzu liegt es im Rahmen der Erfindung, dass die gesamte Fläche des Implantats von einem durchgängigen flächigen Anpassungsbereich ausgefüllt wird, welcher mit einer zusätzlichen Stützstruktur verbunden ist, die in einer zu den Anpassungsbereichen parallelen Ebene verläuft. Damit können unterschiedliche Materialien oder Dicken verwendet werden.

Gemäss einer bevorzugten Ausführungsform weist das Implantat mindestens eine Befestigungslasche zum Befestigen des Implantats an einem Knochen auf. Besonders bevorzugt umfasst mindestens einer der Befestigungslaschen mindestens eine Aufnahme für eine Knochenschraube, mittels der die Befestigung erzielt werden kann. Die Aufnahme, welche vorzugsweise eine die Befestigungslasche durchdringende kreisrunde Öffnung aufweist, ist derart bemessen, dass das Implantat sicher und fest am Knochen fixiert werden kann. Vorteilhafterweise verfügt mindestens eine der Befestigungslaschen mehrere Aufnahmen für Knochenschrauben. Dies ermöglicht einen variablen Einsatz des Implantats, da es durch Auswahl der Aufnahme an die individuelle Anatomie anpassbar ist.

Gemäss einer Weiterbildung ist mindestens eine Befestigungslasche direkt mit der Stützstruktur verbunden. Bevorzugt ist die Befestigungslasche einstückig mit der Stützstruktur verbunden. Dies garantiert eine besonders stabile Fixierung des Implantats am Knochen.

Weiterhin bevorzugt ist mindestens eine Befestigungslasche mit einem Randbereich der Stützstruktur verbunden. Dies führt zu einem besonders festen Halt des Implantats, da bei der Einsetzung des Implantats die Befestigungslaschen in einem von der Knochenfraktur entfernten Knochenbereich angeordnet werden können, welcher stabiler ist als der zum Teil filigrane Orbitabereich.

Gemäss einer besonderen Ausführungsform ist das gesamte Implantat einstückig ausgebildet. Das Implantat wird bevorzugt durch Ätzen aus einem Stück hergestellt.

Bevorzugt weisen zumindest Teile der Stützstruktur einen grösseren Querschnitt als die Anpassungsbereiche auf. Dies kann einerseits bedeuten, dass die Teile der Stützstruktur in einer Richtung senkrecht zur Hauptebene des Implantats eine grössere Dicke als die Anpassungsbereiche aufweisen. Dabei bezeichnet die Hauptebene eine gedachte Ebene, welche sich in die beiden Hauptausdehnungsrichtungen des flächigen Implantats erstreckt. Falls sich das Implantat in einem gekrümmten Zustand befindet, ist die Hauptebene an einem Punkt als die Tangentialebene an das flächige Implantat durch diesen Punkt zu verstehen.

Diese Bauform ermöglicht eine grössere Stabilität der Stützstruktur und damit des gesamten Implantats, da die Kräfte in der Hauptebene besser übertragen werden können.

Alternativ oder zusätzlich liegt gemäss der hier verwendeten Terminologie auch dann ein grösserer Querschnitt der Stützstruktur vor, wenn Teile der Stützstruktur eine grössere Breite in einer Richtung in der Hauptebene aufweisen als Strukturen innerhalb der Anpassungsbereiche, wie etwa Gitterstege in einem Gitterbereich. Auf diese Weise wird eine Verformung innerhalb der Hauptebene noch effektiver verhindert.

Bevorzugt ist die Stützstruktur im Vergleich zu den Anpassungsbereichen weniger biegbar. Insbesondere ist zur plastischen Deformierung der Stützstruktur an sich eine grössere Kraft erforderlich als zur Deformierung der Anpassungsbereiche an sich. Unter der Stützstruktur an sich wird dabei die separierte, von den Anpassungsbereichen abgetrennte Stützstruktur verstanden. Analog wird unter einem Anpassungsbereich an sich ein separierter Anpassungsbereich verstanden, welcher von der Stützstruktur etwa durch Ausschneiden abgetrennt wurde. Insbesondere ist das Implantat in einem deformierten, insbesondere einem von einer ebenen Form verschiedenen Zustand nur durch die Anwendung einer grösseren Kraft plastisch deformierbar, als dies bei einem Vergleichsimplantat erforderlich ist, bei dem allerdings anstelle der Stützstruktur eine Struktur vorhanden ist, welche im Wesentlichen der Struktur der Anpassungsbereiche entspricht. Mit anderen Worten besitzt das Vergleichsimplantat die gleiche Aussenkontur und die gleiche Gitterstruktur, wobei sich letztere jedoch über die gesamte Fläche erstreckt, ohne dass eine Stützstruktur vorhanden ist.

Die genannten, die Biegbarkeit betreffenden Eigenschaften können etwa durch die Formgebung und/oder die Dimensionierung und/oder die verwendeten Materialien der Stützstruktur und der Anpassungsbereiche eingestellt werden.

Diese die Biegbarkeit betreffenden Eigenschaften ermöglichen ein müheloses Vorgehen bei der Anpassung des Implantats beim Einsetzen. In einem ersten Schritt wird dabei die Stützstruktur in eine Anpassungsform deformiert. Diese Anpassungsform ist durch die Anatomie des zu behandelnden Orbitabereichs vorgegeben. Bevorzugt wird das Implantat direkt auf dem defekten Orbitabereich deformiert, was entweder manuell oder mittels geeigneter Instrumente durchgeführt werden kann. Insbesondere, wenn die Stützstruktur im Wesentlichen eindimensionale Bereiche wie etwa Stützstege aufweist, ist in diesem Schritt nur eine zweidimensionale Anpassung nötig, da nur eine einzige Krümmungsrichtung des Knochenmaterials angenähert werden muss. Die Anpassungsbereiche leisten bei diesem ersten Schritt nur einen geringfügigen Widerstand, der die Deformation der Stützstruktur nicht behindert.

In einem zweiten Schritt werden die Anpassungsbereiche durch Ausübung einer Kraft deformiert. Bevorzugt wird dabei auf die Stützstruktur keine direkte Kraft ausgeübt. Wegen der im Wesentlichen zweidimensionalen Struktur der Anpassungsbereiche ist bei diesem Schritt eine dreidimensionale Anpassung an zwei Krümmungsrichtungen nötig, was aber durch die Flexibilität und die plastische Deformierbarkeit der Anpassungsbereiche ermöglicht ist. Aufgrund der geringeren Biegbarkeit der Stützstruktur wird diese bei der Deformierung der Anpassungsbereiche selbst nicht oder lediglich in einem vergleichsweise geringen Masse deformiert.

Bevorzugt liegt das gesamte Implantat in einer ebenen Form vor, aus der heraus es deformiert werden kann. Es ist aber auch denkbar, das Implantat in einer nicht ebenen Form bereitzustellen. Eine solche ebene Form ist etwa für die Herstellung, die Verpackung oder den Versand besonders geeignet. Weiterhin erlaubt es dem behandelnden Arzt die volle Freiheit bei der Wahl der Indikation und der Anpassung des Implantates.

Gemäss einer bevorzugten Ausführungsform weist das Implantat mindestens einen Solltrennbereich auf, entlang dem Teile des Implantats voneinander trennbar sind. Bevorzugt enthält die Stützstruktur mindestens einen der Solltrennbereiche. Dies ermöglicht es, bei der chirurgischen Verwendung optional und nach Bedarf einzelne Teile des Implantats an geeigneten, vordefinierten Stellen abzutrennen. Die Solltrennbereiche können dabei etwa als Solltrennstege ausgebildet sein.

Gemäss einer besonderen Weiterbildung wurde die Form der Stützstruktur mit Hilfe einer Topologieoptimierung konstruiert. Die Topologieoptimierung ist ein etwa aus dem Maschinen- und Fahrzeugbau an sich bekanntes, computergestütztes Verfahren zur Optimierung eines Bauteils hinsichtlich seiner Gestalt in Bezug auf die auftretenden Kräfte. Insbesondere wird dabei eine Gewichtsverringerung bei maximaler Steifigkeit angestrebt. Zur rechnerischen Modellierung kann unter anderem auf die Methode der Finiten Elemente zurückgegriffen werden. Einen Überblick über diese Technologie liefert beispielsweise das Lehrbuch "Optimierung mechanischer Strukturen - Grundlagen und industrielle Anwendungen" von Axel Schumacher, erschienen beim Springer-Verlag im September 2004.

Eine topologieoptimierte Struktur verleiht dem erfindungsgemässen Implantat eine aussergewöhnliche Steifigkeit gegenüber Verbiegungen und Verzerrungen in der Implantatebene bei gleichzeitiger Material- und damit Gewichtseinsparung. Demgemäss werden bei der Topologieoptimierung eine Optimierung der Steifigkeit und/oder eine Minimierung des Volumens des Implantats angestrebt. Dabei werden gleichzeitig beispielsweise die Dicke des Implantats senkrecht zu seiner Hauptebene und/oder das Volumenverhältnis zwischen Material und Hohlräumen und/oder die Materialdaten und/oder die Randbedingungen wie etwa die Position der Befestigungslaschen festgehalten.

Bevorzugt hat das Implantat eine Dicke im Bereich von 0,1 bis 4 mm, bevorzugt von 0,1 bis 1 mm, besonders bevorzugt von 0,1 bis 0,3 mm. Diese Dicke wird dabei senkrecht zu einer Hauptebene des Implantats bestimmt. Weiterhin ist es derart dimensioniert, dass es in eine Augenhöhle, insbesondere in eine menschliche Augenhöhle, einsetzbar ist.

Die Form zumindest eines Teilbereichs des Implantats kann topologieoptimiert sein. Die Begriffe "Implantat" und "topologieoptimiert" werden hierbei wie oben verstanden. Insbesondere kann ein Teil einer Stützstruktur topologieoptimiert sein. Eine Stützstruktur bezeichnet allgemein einen Teil des Implantats, welcher weniger biegsam ist als andere Teile des Implantats und/oder einen wesentlichen Beitrag zur Steifigkeit des Implantats liefert.

Offenbart ist auch ein Verfahren zum Konstruieren der Form eines Implantats, in dem die Form wenigstens eines Teilbereichs des Implantats mit Hilfe einer Topologieoptimierung definiert wird. Insbesondere kann es sich bei dem Teil des Implantats um eine Stützstruktur des Implantats handeln.

Schliesslich ist eine Stützstruktur für ein Implantat offenbart, bei dem die Form wenigstens eines Teils topologieoptimiert ist. Derartige Stützstrukturen können je nach Bedarf mit an sich bekannten Anpassungsbereichen, wie etwa bekannten Gitterbereichen, versehen werden.

Im Folgenden wird die Erfindung anhand von Ausführungsbeispielen und mit Hilfe von Figuren erläutert. Es zeigen:
- Fig. 1: eine Draufsicht auf eine erste Ausführungsform eines Implantats gemäss der vorliegenden Erfindung;
- Fig. 2: die Stützstruktur des Implantats gemäss Fig. 1;
- Fig. 3: eine Draufsicht auf eine zweite Ausführungsform eines Implantats gemäss der vorliegenden Erfindung;
- Fig. 4: die Stützstruktur des Implantats gemäss Fig. 3;
- Fig. 5: ein in eine Orbita eingesetztes Implantat.

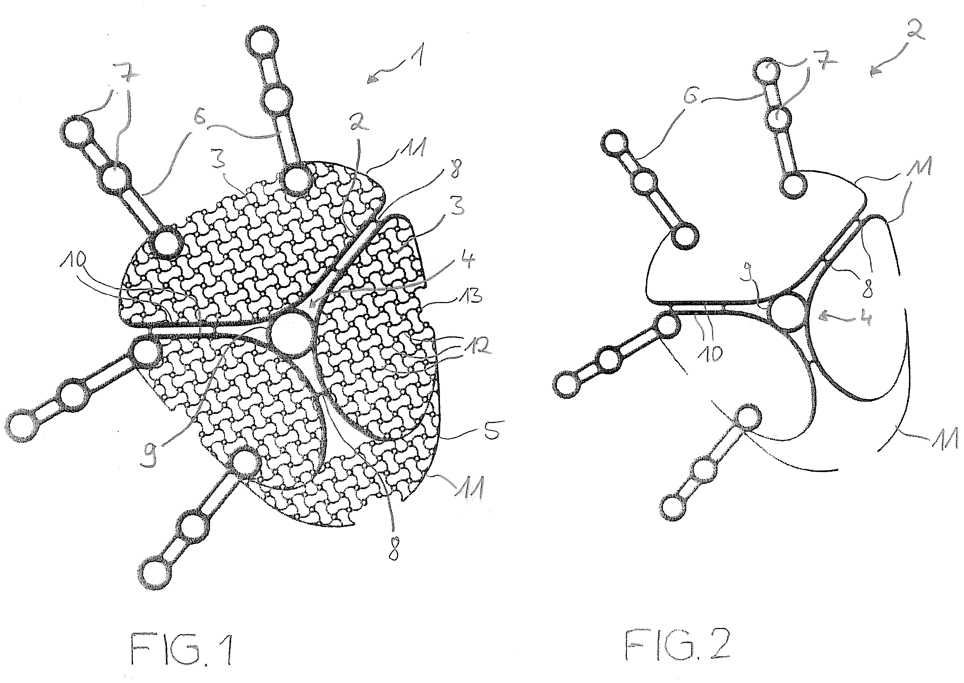

Das in Figur 1 dargestellte erfindungsgemässe Implantat 1 weist eine Stützstruktur 2 und vier flächige als Gitterbereiche 3 ausgebildete Anpassungsbereiche auf. Die Hauptebene des flachen, aber deformierbaren, Implantats 1 stimmt in dieser Darstellung mit der Zeichenebene überein. Die Stützstruktur 2 weist einen Innenbereich 4 auf, der innerhalb eines Randes 5 des Implantats 1 angeordnet ist. (Für eine separate Darstellung der Stützstruktur 2 s. a. Figur 2.) Der Innenbereich 4 verfügt über einen Zentralbereich, der als Ring 9 ausgebildet ist. Weiterhin weist der Innenbereich 4 sechs Stützstege 10 auf, welche sich vom Zentralbereich 9 in Richtung des Randes 5 des Implantats 1 erstrecken. Jeweils zwei der Stützstege 10 verlaufen im Wesentlichen parallel zueinander und sind über mindestens einen als Solltrennsteg 8 ausgebildeten Solltrennbereich verbunden. Einer oder mehrere der Solltrennstege 8 können je nach Defekt und im Ermessen des behandelnden Arztes durchtrennt werden. Jeweils zwei Paare von Stützstegen 10 schliessen einen Winkelbereich von etwa 120 ° aus. Die Solltrennstege 8 ermöglichen es, Teile des Implantats 1 voneinander an vordefinierten Stellen kontrolliert und sauber abzutrennen. Die Stützstege 10 sind mit Randbereichen 11 der Stützstruktur 2 verbunden, welche in dieser Ausführungsform etwa 80 % des Randes 5 des Implantats 1 bilden. Alternativ hierzu ist es denkbar und liegt im Rahmen der Erfindung, dass die Randbereiche 11 das Implantat 1 vollständig umschliessen und somit den kompletten Rand 5 des Implantats 1 bilden.

Die Form der gesamten Stützstruktur 2 wurde mit Hilfe einer Topologieoptimierung konstruiert. Dazu wurden eine Optimierung der Steifigkeit und eine Minimierung des Volumens des Implantats angestrebt. Gleichzeitig wurden die Dicke des Implantats senkrecht zu seiner Hauptebene, das Volumenverhältnis zwischen Material und Hohlräumen, die Materialdaten und der Rand (5) festgehalten.

Die Gitterbereiche 3 sind durch den Zentralbereich 9 und die Stützstege 10 und somit durch Teile der Stützstruktur 2 räumlich voneinander getrennt. Die Gitterbereiche 3 weisen periodische Strukturen auf und umfassen eine Vielzahl gebogener Gitterstege 12, von denen im Inneren des Gitters jeweils vier an einem gemeinsamen Gitterring 13 zusammenlaufen. Andere Gitterstrukturen wären aber im Rahmen der Erfindung ebenso denkbar.

Die Stützstege 10, welche Teile der Stützstruktur 2 bilden, weisen in der Hauptebene, die mit der Zeichenebene übereinstimmt, eine Breite von etwa 0,5 mm auf, während die Gitterstege 12 lediglich eine Breite von etwa 0,2 mm aufweisen. Aufgrund dessen ist die Stützstruktur 2 weniger biegbar als die Gitterbereiche 3, was den Einsatz des Implantats 1 wie oben beschrieben erleichtert. Die Randbereiche 11 haben in etwa die gleiche Breite wie die Gitterstege 12.

Weiterhin sind die Stützstege 10 und die Randbereiche 11 der Stützstruktur 2 senkrecht zur Zeichenebene - also senkrecht zur Hauptebene des Implantats 1 - dicker ausgebildet als die Gitterbereiche 3. Die Dicke der Gitterbereiche 3 senkrecht zur Zeichenebene beträgt etwa 0,15 mm, die Dicke der Stützstruktur 2 senkrecht zur Zeichenebene beträgt etwa 0,2 mm. Auch dies trägt dazu bei, dass die Stützstruktur 2 weniger biegbar ist als die Gitterbereiche 3.

Am Randbereich 11 der Stützstruktur 2 sind insgesamt vier Befestigungslaschen 6 angeordnet und damit einstückig verbunden. Dabei sind zwei der Befestigungslaschen 6 am freien Ende eines Randbereichs 11 angeordnet, während die beiden anderen Befestigungslaschen 6 an Teilen eines Randbereichs 11 angeordnet sind, an denen diese auf die Stützstege 10 treffen. Die Befestigungslaschen 6 weisen jeweils drei Aufnahmeöffnungen 7 für jeweils eine hier nicht dargestellte Knochenschraube auf, wobei jeweils eine der Aufnahmeöffnungen 7 in der konvexen Hülle der Gitterbereiche 3 angeordnet ist. Die Aufnahmeöffnungen 7 haben einen Durchmesser von etwa 1,9 mm. Sie weisen bevorzugt keine Einsenkungen auf. Somit kann das Implantat 1 sowohl für die rechte als auch die linke Orbita verwendet werden, indem das Implantat bei Bedarf um einen Winkel von 180° um eine in der Zeichenebene liegende Achse gedreht wird. Durch Einsetzen von Knochenschrauben in alle oder einen Teil der Aufnahmeöffnungen 7 und Eindrehen in ein Knochenmaterial lässt sich somit das Implantat am Knochenmaterial fixieren.

Das gesamte Implantat 1 ist aus Titan gefertigt und einstückig ausgebildet. Bevorzugt wird es mittels Ätzen hergestellt. Es kann für so genannte Frakturtypen 1-2 genutzt werden (vgl. die "Classification of orbital wall defects" gemäss B. Hammer).

Die Figur 2 zeigt separat die Stützstruktur 2 des Implantats gemäss Figur 1. Die Form der Stützstruktur 2 wurde mit Hilfe einer Topologieoptimierung bestimmt; für weitere Details hierzu wird auf die obigen Erläuterungen verwiesen. Die hier dargestellte Stützstruktur 2 ist nicht mit Gitterbereichen verbunden und kann daher einerseits als ein Zwischenprodukt bei der Herstellung eines erfindungsgemässen Implantats 1 angesehen werden. Andererseits ist auch der direkte Einsatz einer solchen Stützstruktur 2 mit optionalen Befestigungslaschen 6 als Implantat denkbar, falls Gitterbereiche wie im ersten Aspekt der Erfindung nicht benötigt werden.

Weiterhin ist ein Einsatz einer solchen Stützstruktur 2 in Kombination mit einem Material denkbar, welches einen ähnlichen Zweck erfüllt wie das Material der Anpassungsbereiche, wie z.B. eine Kunststoff-Folie. Eine solche Ausführungsform ist daher auf jeden Fall zumindest zweistückig.

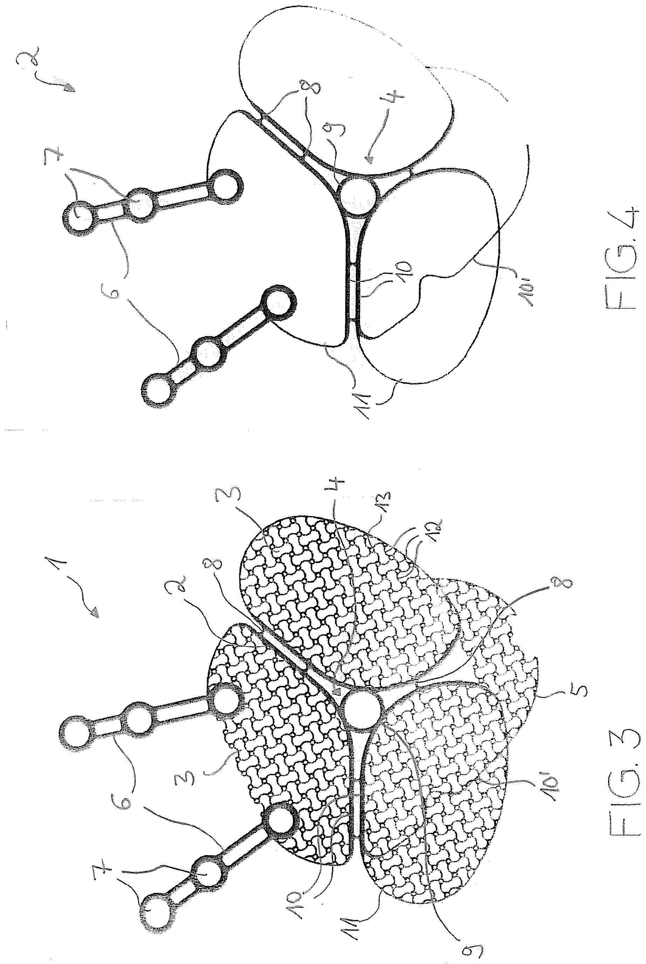

Eine zweite mögliche Ausführungsform des erfindungsgemässen Implantats 1 ist in Figur 3 wiedergegeben. Im Gegensatz zur ersten Ausführungsform weist dieses Implantat 1 lediglich zwei Befestigungslaschen 6 auf. Überdies verfügt es über einen weiteren Stützsteg 10', der einen Teil des Innenbereichs 4 der Stützstruktur 2 bildet, sich aber nicht vom Zentralbereich 9 erstreckt. Ein Implantat 1 in dieser zweiten Ausführungsform kann bei so genannten Frakturtypen 2-4 Verwendung finden (Classification of orbital wall defects gemäss B. Hammer). Figur 4 zeigt separat die Stützstruktur 2 des Implantats 1 aus Figur 3.

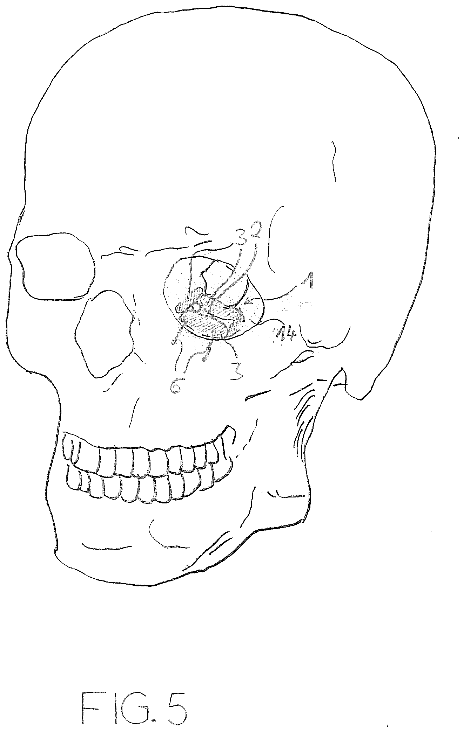

Die Figur 5 zeigt ein in eine Orbita 14 eingesetztes erfindungsgemässes Implantat 1 aus Figur 1. Die Stützstruktur 2 sowie ein Grossteil der Gitterbereiche 3 liegen am Orbitaboden 15 auf. Zwei der Befestigungslaschen 6 ragen aus der Orbita 14 hinaus und sind am Wangenknochen angeordnet. Die beiden weiteren Befestigungslaschen, welche in der Figur 1 dargestellt sind, wurden vor dem Einsetzen vom Rest des Implantats abgetrennt.

## Patentansprüche

1. Implantat (1) zur Behandlung von Knochen, insbesondere zur Abdeckung von Defekten oder Bohrlöchern oder zur Rekonstruktion von Knochendefekten und Fehlbildungen, insbesondere von Frakturen eines Orbitabodens, umfassend
- mindestens einen insbesondere flächigen Anpassungsbereich (3),
- wenigstens eine Befestigungslasche (6) zum Befestigen des Implantats (1) an einem Knochen und
- eine Stützstruktur (2), welche einen Innenbereich (4) aufweist, der innerhalb eines Randes (5) des Implantats (1) angeordnet ist, wobei
- der Anpassungsbereich (3) plastisch deformierbar ist,
**dadurch gekennzeichnet, dass**
- die Stützstruktur (2) im Vergleich zu dem mindestens einen Anpassungsbereich (3) weniger biegbar ist.

2. Implantat (1) gemäss Anspruch 1,
**dadurch gekennzeichnet, dass**
der Innenbereich (4) der Stützstruktur (2) einen Zentralbereich (9) umfasst, von welchem sich mindestens ein Stützsteg (10, 10'), bevorzugt mindestens drei Stützstege (10, 10'), in Richtung des Randes (5) des Implantats (1) erstrecken.

3. Implantat (1) gemäss einem der Ansprüche 1 und 2,
**dadurch gekennzeichnet, dass**
die Stützstruktur (2) Randbereiche (11) aufweist, welche teilweise oder ganz den Rand (5) des Implantats (1) bilden.

4. Implantat (1) gemäss einem der Ansprüche 1 bis 3,
**dadurch gekennzeichnet, dass**
das Implantat (1) zumindest zwei Anpassungsbereiche (3) aufweist, welche durch einen Teil des Innenbereichs (4) voneinander räumlich getrennt sind.

5. Implantat (1) gemäss einem der Ansprüche 1 bis 4,
**dadurch gekennzeichnet, dass**
wenigstens eine der Befestigungslaschen (6) mindestens eine Aufnahme (7) für eine Knochenschraube aufweist.

6. Implantat (1) gemäss einem der Ansprüche 1 bis 5,
**dadurch gekennzeichnet, dass**
mindestens eine Befestigungslasche (6) insbesondere einstückig mit der Stützstruktur (2), insbesondere mit einem Randbereich (11), verbunden ist.

7. Implantat (1) gemäss einem der Ansprüche 1 bis 6,
**dadurch gekennzeichnet, dass**
es einstückig ausgebildet ist.

8. Implantat (1) gemäss einem der Ansprüche 1 bis 7,
**dadurch gekennzeichnet, dass**
mindestens ein Anpassungsbereich (3) als Gitterbereich ausgebildet ist.

9. Implantat (1) gemäss einem der Ansprüche 1 bis 8,
**dadurch gekennzeichnet, dass**
es ein biokompatibles Material, insbesondere Titan und/oder ein biokompatibles polymeres oder polymer-keramisches Material, umfasst.

10. Implantat (1) gemäss einem der Ansprüche 1 bis 9,
**dadurch gekennzeichnet, dass**
die Stützstruktur (2) derart ausgebildet und angeordnet ist, dass das Implantat (1) in eine vordefinierte Form insbesondere dreidimensional deformierbar ist.

11. Implantat (1) gemäss einem der Ansprüche 1 bis 10,
**dadurch gekennzeichnet, dass**
zumindest Teile der Stützstruktur (2)
- in einer Richtung senkrecht zu einer Hauptebene des Implantats (1) eine grössere Dicke als die Anpassungsbereiche (3) aufweisen und/oder
- eine grössere Breite in einer Richtung in der Hauptebene des Implantats (1) aufweisen als Strukturen innerhalb der Anpassungsbereiche (3), insbesondere als Gitterstege (12) in einem Gitterbereich.

12. Implantat (1) gemäss einem der Ansprüche 1 bis 11,
**dadurch gekennzeichnet, dass**
das Implantat (1), insbesondere die Stützstruktur (2) mindestens einen Solltrennbereich (8) aufweist, entlang dem Teile des Implantats (1) voneinander trennbar sind.

13. Implantat (1) gemäss einem der Ansprüche 1 bis 12,
**dadurch gekennzeichnet, dass**
die Form und/oder Dimensionierung der Stützstruktur (2) mit Hilfe einer Topologieoptimierung konstruiert wurde.

14. Implantat (1) gemäss einem der Ansprüche 1 bis 13,
**dadurch gekennzeichnet, dass**
es in einer Richtung senkrecht zu einer Hauptebene des Implantats (1) eine Dicke im Bereich von 0,1 bis 4 mm, bevorzugt von 0,1 bis 1 mm, besonders bevorzugt von 0,1 bis 0,3 mm aufweist.

## Claims

1. Implant (1) for treating bones, in particular for covering defects or boreholes or for repairing bone defects and malformations, in particular fractures of an orbital floor, comprising
- at least one in particular planar adaptation region (3),
- at least one fastening tab (6) for fastening the implant (1) to a bone, and
- a support structure (2) which has an inner region (4) arranged inside an edge (5) of the implant (1),
wherein
- the adaptation region (3) is plastically deformable,
**characterized in that**
- the support structure (2) is less bendable than the at least one adaptation region (3).

2. Implant (1) according to Claim 1, **characterized in that** the inner region (4) of the support structure (2) comprises a central region (9) from which at least one support web (10, 10'), preferably at least three support webs (10, 10'), extend(s) in the direction of the edge (5) of the implant (1).

3. Implant (1) according to either of Claims 1 and 2, **characterized in that** the support structure (2) has edge regions (11) which partially or completely form the edge (5) of the implant (1).

4. Implant (1) according to one of Claims 1 to 3, **characterized in that** the implant (1) has at least two adaptation regions (3) which are spatially separated from each other by a part of the inner region (4).

5. Implant (1) according to one of Claims 1 to 4, **characterized in that** at least one of the fastening tabs (6) has at least one seat (7) for a bone screw.

6. Implant (1) according to one of Claims 1 to 5, **characterized in that** at least one fastening tab (6) is connected in particular integrally to the support structure (2), in particular to an edge region (11).

7. Implant (1) according to one of Claims 1 to 6, **characterized in that** it is formed in one piece.

8. Implant (1) according to one of Claims 1 to 7, **characterized in that** at least one adaptation region (3) is configured as a lattice region.

9. Implant (1) according to one of Claims 1 to 8, **characterized in that** it comprises a biocompatible material, in particular titanium and/or a biocompatible polymeric or polymeric-ceramic material.

10. Implant (1) according to one of Claims 1 to 9, **characterized in that** the support structure (2) is configured and arranged in such a way that the implant (1) is deformable, in particular three-dimensionally, to a predefined shape.

11. Implant (1) according to one of Claims 1 to 10, **characterized in that** at least parts of the support structure (2) have
- a greater thickness than the adaptation regions (3) in a direction perpendicular to a main plane of the implant (1), and/or
- a greater width, in a direction in the main plane of the implant (1), than structures inside the adaptation regions (3), in particular than lattice webs (12) in a lattice region.

12. Implant (1) according to one of Claims 1 to 11, **characterized in that** the implant (1), in particular the support structure (2), has at least one predetermined separation region (8) along which parts of the implant (1) are separable from each other.

13. Implant (1) according to one of Claims 1 to 12, **characterized in that** the shape and/or dimensions of the support structure (2) have been designed with the aid of topology optimization.

14. Implant (1) according to one of Claims 1 to 13, **characterized in that**, in a direction perpendicular to a main plane of the implant (1), it has a thickness in the range from 0.1 to 4 mm, preferably from 0.1 to 1 mm, particularly preferably from 0.1 to 0.3 mm.

## Revendications

1. Implant (1) destiné au traitement de l'os et en particulier à couvrir des défauts ou des trous forés ou à reconstruire des défauts de l'os et des malformations, en particulier des fractures du fond de l'orbite, l'implant comprenant :
- une ou plusieurs parties d'adaptation (3), notamment plate,
- une ou plusieurs pattes de fixation (6) servant à fixer l'implant (1) sur un os et
- une structure de soutien (2) qui présente une partie intérieure (4) disposée à l'intérieur d'un bord (5) de l'implant (1),
- la partie d'adaptation (3) étant déformable plastiquement,
**caractérisé en ce que**
- la structure de soutien (2) est moins flexible que la ou les parties d'adaptation (3).

2. Implant (1) selon la revendication 1, **caractérisé en ce que** la partie intérieure (4) de la structure de soutien (2) comporte une partie centrale (9) depuis laquelle une ou plusieurs nervures de soutien (10, 10'), de préférence trois nervures de soutien (10, 10'), s'étendent en direction du bord (5) de l'implant (1).

3. Implant (1) selon l'une des revendications 1 et 2, **caractérisé en ce que** la structure de soutien (2) présente des parties de bord (11) qui forment tout ou partie du bord (5) de l'implant (1).

4. Implant (1) selon l'une des revendications 1 à 3, **caractérisé en ce que** l'implant (1) présente deux ou plusieurs parties d'adaptation (3) séparées les unes des autres par une partie de la partie intérieure (4).

5. Implant (1) selon l'une des revendications 1 à 4, **caractérisé en ce qu'**au moins l'une des pattes de fixation (6) présente un ou plusieurs logements (7) pour vis à os.

6. Implant (1) selon l'une des revendications 1 à 5, **caractérisé en ce qu'**au moins l'une des pattes de fixation (6) est reliée en particulier d'un seul pièce à la structure de soutien (2) et en particulier à une implant (11).

7. Implant (1) selon l'une des revendications 1 à 6, **caractérisé en ce qu'**il est formé d'une seule pièce.

8. Implant (1) selon l'une des revendications 1 à 7, **caractérisé en ce qu'**une ou plusieurs parties d'adaptation (3) sont configurées comme parties en grille.

9. Implant (1) selon l'une des revendications 1 à 8, **caractérisé en ce qu'**il comporte un matériau biocompatible, notamment le titane et/ou un polymère biocompatible ou un matériau en polymère-céramique.

10. Implant (1) selon l'une des revendications 1 à 9, **caractérisé en ce que** la structure de soutien (2) est configurée et disposée de telle sorte que l'implant (1) soit déformable en une forme prédéfinie et en particulier tridimensionnelle.

11. Implant (1) selon l'une des revendications 1 à 10, **caractérisé en ce qu'**une ou plusieurs parties de la structure de soutien (2)
- présentent une épaisseur supérieure à celle des parties d'adaptation (3) dans une direction perpendiculaire à un plan principal de l'implant (1) et/ou
- présentent dans la direction d'un plan principal de l'implant (1) une largeur supérieure à celle de structures situées à l'intérieur des parties d'adaptation (3) et en particulier de nervures de grille (12) d'une partie en grille.

12. Implant (1) selon l'une des revendications 1 à 11, **caractérisé en ce que** l'implant (1) et en particulier la structure de soutien (2) présentent une ou plusieurs parties (8) de rupture préférentielle suivant lesquelles des parties de l'implant (1) peuvent être séparées les unes des autres.

13. Implant (1) selon l'une des revendications 1 à 12, **caractérisé en ce que** la forme et/ou les dimensions de la structure de soutien (2) sont définies à l'aide d'une optimisation topologique.

14. Implant (1) selon l'une des revendications 1 à 13, **caractérisé en ce que** dans une direction perpendiculaire à un plan principal de l'implant (1), il présente une épaisseur de l'ordre de 0,1 à 4 mm, de préférence de 0,1 à 1 mm et de façon particulièrement préférable de 0,1 à 0,3 mm.
